# EUROPEAN PATENT APPLICATION

(11) **EP 4 311 540 A1**
(43) Date of publication of application: **31.01.2024**
(21) Application number: 23187155.9
(22) Date of filing: 24.07.2023
(51) Int. Cl.: A61K 9/00, A61K 31/095, A61K 31/198, A61K 33/04, A61K 36/28, A61K 36/899, A61K 38/06, A61P 1/16, A61K 31/695

(54) **EDIBLE HERBAL COMPOSITION CONTAINING SILICON, AND PROCEDURE FOR OBTAINING IT**

(30) Priority: 27.07.2022 IT 202200015906
(71) Applicant: Carepharm S.r.l., Pieve al Toppo - Civitella in Val di Chiana (AR) (IT)
(72) Inventor: DE ANGELIS, Carlo, 52041 PIEVE AL TOPPO - CIVITELLA IN VAL DI CHIANA (AR) (IT); GIOVANNONE, Daniele, 52041 PIEVE AL TOPPO - CIVITELLA IN VAL DI CHIANA (AR) (IT)
(74) Representative: Bianchetti & Minoja with Trevisan & Cuonzo IPS SRL

(57) **Abstract**

Disclosed is a composition of botanical extracts and active ingredients comprising glutathione and silicon, and pharmaceutical, nutraceutical or veterinary formulations containing it. The preferred botanical extracts are artichoke extract and milk thistle extract. Silicon, present as orthosilicic acid, preferably deriving from plant sources, increases the bioavailability of the botanical extracts without interfering with the absorption of the other ingredients of the combination.

## Description

The invention relates to a composition of botanical extracts and active ingredients comprising glutathione and silicon, and pharmaceutical, nutraceutical or veterinary formulations containing it.

### Background of the invention

The liver is particularly liable to states of altered operation, which can degenerate into diseases. In its normal physiological role, the liver is liable to accumulate toxic substances (or cell metabolism waste substances) up to high levels, which may become pathological. High alcohol consumption in particular can cause liver disease, but pathological states unrelated to alcohol consumption also exist. In the event of mild alterations of the liver metabolism, enlargement of the liver may be observed, a state usually described as "fatty liver". Unless suitably treated, said state can degenerate into a disease, namely cirrhosis.

In addition to specific medicaments indicated for various liver disorders, there are numerous preparations on the market which can help the liver to perform its detoxifying action; they are typically herbal or nutraceutical preparations, often based on long experience of use.

Artichoke (*Cynara spp)* and milk thistle *(Silybum marianum)* extracts have long been used for therapeutic and health purposes in the treatment of liver disorders of various origins and severities (viral hepatitis, poisoning, steatosis and cirrhosis).

They are marketed as preparations for oral use, for example in the form of preparations for herbal teas, or in capsule or tablet form, variously combined with other active ingredients. The main active constituents of said extracts, in particular cynarin in artichoke extract and silibinin (silybin) and correlated compounds (silydianin, silychristin or mixtures thereof such as silymarin) in milk thistle extract do not have satisfactory bioavailability. In the case of milk thistle, an adverse effect exerted by various metals on intestinal absorption of silibinin is also known. In an attempt to obviate said drawbacks, the use of phospholipids of plant origin able to form plant compounds that improve its absorption has been proposed.

However, the use of herbal preparations is not wholly satisfactory, mainly due to two types of problems: (i) the active ingredient content is highly variable, depending on the geographical origin of the plant, the harvesting period and numerous other variables, and (ii) the administration of an active ingredient does not correspond to its complete absorption. In the case of the active ingredients of milk thistle, an adverse effect exercised by various metals on the intestinal absorption of silibinin is known.

Glutathione (GSH) is a natural compound normally present in animal cells, and involved in numerous biochemical processes. Physiologically, it has three different roles: a) detoxifying, b) metal chelating and c) antioxidant and reducing. It is involved in the health of the liver, wherein poorly water-soluble organic toxins are converted to highly water-soluble glutathionyl derivatives, which are then eliminated with the urine. Moreover, in view of its reducing properties, it reduces some of the side effects of alcohol consumption. In the event of increased demand, glutathione is administered as an injectable medicament; oral administration is not very effective because GSH is largely destroyed in the gastrointestinal tract. A possible alternative is the use of N-acetylcysteine (NAC), a precursor of glutathione.

Glutathione can be used to treat non-alcoholic fatty liver disease, as described in US11207371.

Diet compositions comprising various plant extracts and silicon dioxide are present on the market. The latter ingredient is used as an excipient, and does not constitute a bioavailable silicon source. See, in this respect, British Journal of Nutrition (2009), 102, 825-834 and Handbook of Pharmaceutical Excipients, 6th Edition, Edited by Raymond C Rowe, Paul J Sheskey BSc, RPh, Marian E Quinn, page 185, and British Journal of Nutrition (2009), 102, 825-834.

EP 33456223 discloses compositions containing a number of ingredients, including artichoke, nettle and *Silybum marianum* extracts. Nettle extract is not identified as a possible source of bioavailable silicon, and glutathione is not mentioned.

CH 714 140 discloses compositions comprising collagen, astaxanthin and extracts of plants including artichoke and *Silybum marianum.* The optional ingredients mentioned include orthosilicic acid, but not glutathione.

WO2013 discloses a composition for the treatment of hepatitis C comprising various extracts or seeds of plants including nettle, artichoke and milk thistle. Silicon and glutathione are not mentioned.

The present invention solves the problems of incompatibility and non-absorption of the active ingredients described, and improves their intestinal absorption.

### Description of the invention

It has been found that the administration of an absorbable form of silicon has favourable effects on the intestinal absorption of cynarin without interfering with the absorption of glutathione or N-acetylcysteine (NAC) or other botanical preparations, in particular silybin absorption; the use of silicon therefore allows simultaneous administration of artichoke, milk thistle and glutathione extracts.

In the nutritional field, diet supplements for sports players based on orthosilicic acid, claimed to support cardiac activity during and after training, are marketed in the USA. A combination of orthosilicic acid and vanillin has also been prepared, which claims to solve the problem of stability of orthosilicic acid and is proposed as an absorbable form of silicon.

Silicon is present in nearly all plant-based foods in a poorly absorbable form; of the plants included in traditional herbalism, nettle (Urtica dioica), horsetail (Equisetum spp.) and bamboo are possible sources of bioavailable silicon. Some formulations of bioavailable silicon are available on the market, such as plant extracts of rosemary, horsetail or aloe.

It has now been experimentally observed that combining a source of absorbable silicon with a preparation based on dried artichoke extract increases cynarin absorption, but does not interfere with the absorption of other active ingredients of artichoke. The same source of absorbable silicon does not adversely interfere with the absorption of glutathione, NAC, zinc or the active ingredients of milk thistle, and can therefore be combined with said ingredients.

The object of the invention is therefore dietary or oral pharmaceutical compositions comprising cynarin or an extract containing it, glutathione or a precursor thereof, and a plant extract comprising bioavailable silicon.

The compositions can also contain silybin or an extract containing it, in particular a milk thistle extract.

The extract containing cynarin is preferably an artichoke (*Cynara scolymus)* leaf extract, in particular a dried extract of artichoke leaves, and the glutathione is preferably reduced glutathione, more preferably reduced glutathione in crystalline form. N-acetylcysteine is a glutathione precursor.

The extract comprising bioavailable silicon is preferably a bamboo shoot extract with a 70% silicon content.

The unit doses of the ingredients of the compositions according to the invention can range within wide limits, determinable by the skilled person on the basis of the known properties of artichoke and thistle extracts and GSH.

Broadly speaking, the unit doses per dose administered can range from 50 to 500 mg for artichoke extracts, glutathione (reduced glutathione or NAC) and milk thistle, and 10 to 100 mg for the bioavailable silicon source (orthosilicic acid or plant extract containing it, in particular a bamboo shoot or leaf extract). A specific composition contains 200 mg of glutathione, 200 mg of artichoke extract, 200 mg of milk thistle extract and 20 mg of silicon source.

The compositions according to the invention can also comprise other active ingredients useful for detoxification, such as a selenium source, preferably L-selenomethionine.

Artichoke and milk thistle extracts with various cynarin and silybin contents respectively are available on the market from several sources.

The compositions according to the invention can be prepared in the form of capsules, tablets, granulates, soft gels, solutions, suspensions and syrups, using conventional technological excipients and ingredients such as glidants, disintegrating agents, surfactants, lubricants, sweeteners, phospholipids and flavourings.

The preferred forms are uncoated or film-coated tablets or orally dissolving granulate which can be formulated in capsules or stick-packs.

The following examples illustrate the invention in more detail.

### Example 1: Active ingredient absorption test

The absorption of the active ingredients cynarin, silibinin and glutathione was evaluated with a reconstructed human oral mucosa model to simulate absorption in the oral cavity, and an intestinal epithelium model to simulate gastrointestinal absorption. The studies were conducted by evaluating the passage of the active ingredients through a cell layer (HOE cells for the oral mucosa and CACO-2 cells for the enterocytes), using a well system; the active ingredients were added to the apical compartment only, and the concentration of said active ingredients in both the apical and the basolateral compartments was evaluated after a suitable time. At the beginning of the experiments the integrity of the cell layer was examined under the microscope, discarding wells which were not intact, and the cell viability was checked by MTS assay with tetrazolium yellow, discarding wells with less than 95% viable cells. At the end of the experiments the integrity and viability were checked again, with the results set out below. The experiments were conducted for 5 and 10 minutes in the case of simulated oral absorption (HOE cells), and for 30 and 60 minutes in the case of simulated intestinal absorption (CACO2 cells). At the end of each experiment fluid was drawn from the apical compartment and the basolateral compartment, evaluating its volume and analysing the active ingredient content by semiquantitative TLC.

### Example 2: Simulated intestinal absorption

Various mixtures were prepared, with the ingredients in amounts as described in the table below to evaluate their intestinal absorption as described in the preceding example.

Glutathione used was pure reduced powdered glutathione, amounting to 200 mg per dose. The artichoke used was 200 mg per dose of dried artichoke (*Cynara scolymus)* cauline leaf extract with a chlorogenic acid content of not less than 2.5% (corresponding to an extraction ratio of about 5:1). For the metals, copper (II) citrate, iron (II) fumarate, iron (III) oxide, zinc citrate, titanium dioxide and selenomethionine were assayed. In the table, for all the metals and silicon the content is expressed as ion, in milligrams per dose (micrograms per dose in the case of selenium). Silicon was used both in the form of SiO₂ and in the form of orthosilicic acid in aqueous solution.

Simulated absorption of glutathione and of the active ingredients of artichoke was evaluated compared with composition R (containing only glutathione and dried artichoke extract), expressed as the amount of active ingredient present from the basolateral side compared with the dose administered on the apical side.

**Table 1 Compositions in powder form**

| Sample | Cu⁺⁺ | Se⁺⁺ | Fe⁺⁺ | Fe³⁺ | Zn⁺⁺ | SiO₂ | H₄SiO₄ | Cynarin |
|---|---|---|---|---|---|---|---|---|
| R | - | - | - | | - | - | - | 10% |
| A | 20 mg | - | - | | - | - | - | 10% |
| B | - | 60 mg | - | | - | - | - | 10% |
| C | - | - | 10 mg | 10 mg | - | - | - | 5% |
| D | - | - | - | | 20 mg | - | - | 5% |
| E | - | - | - | | - | 20 mg | - | 10% |
| F | - | - | - | | - | - | 20 mg | 40% |

Formulations A-F were used for simulated absorption tests as described in experiment 1, evaluating the concentration of chlorogenic acid, ferulic acid, cynarin, reduced glutathione and oxidised glutathione in the basolateral compartment of the wells.

The cynarin content is shown in the table, oxidised glutathione was practically nil in all samples, and no differences were observed for any of the other active ingredients.

The data observed demonstrate that unlike reference composition R, selenium, copper, titanium dioxide and silicon dioxide do not interfere with cynarin absorption, whereas the presence of iron and zinc has a modest adverse effect. In the case of the sample containing orthosilicic acid, a surprising increase in (simulated) cynarin absorption was observed, but no effect on the other active ingredients.

### Example 3: Further formulations comprising silicon in various forms

To clarify the role of orthosilicic acid in the absorption of the formulations of the preceding example, further formulations containing silicon were prepared, with special attention to the most absorbable forms. In particular the following were used: silicon dioxide, orthosilicic acid, orthosilicic acid complex with vanillin, silicon complex with choline, and bamboo leaf extract titrated in silicon. The table shows the silicon content in mg/dose. In the absorption test, only the cynarin concentration in the basolateral well was evaluated.

**Table 2 Compositions in powder form**

| | Artichoke, dried extract | Glutathione | SiO₂ | H₄SiO₄ | Si-van | Si-col | Bamboo, dried extract | Cynarin |
|---|---|---|---|---|---|---|---|---|
| R | 200 | 200 | - | - | - | - | - | 10% |
| G | 200 | 200 | 40 | - | - | - | - | 10% |
| H | 200 | 200 | - | 40 | - | - | - | 40% |
| I | 200 | 200 | - | - | 40 | - | - | 40% |
| L | 200 | 200 | - | - | - | 40 | - | 40% |
| M | 200 | 200 | - | | - | - | 40 | 45% |
| N | 200 | - | - | - | - | - | 40 | 45% |
| O | 100 | - | - | - | - | - | 10 | 45% |
| P | 400 | - | - | - | - | - | - | 5% |
| Q | 400 | - | - | - | - | - | 40 | 25% |

The results set out above demonstrate that the administration of orthosilicic acid, either free or complexed with an organic part, has a favourable effect on simulated absorption of cynarin, which increases about four-fold compared with silicon dioxide or the silicon-free formulation (sample R). The effect is evident at doses ranging from 100 to 400 mg of cynarin, and is independent of the presence of glutathione.

### Example 4: Multi-ingredient formulations

This test evaluated whether silicon interferes in any way with simulated intestinal absorption of other active ingredients of pharmaceutical interest for the formulations designed for liver treatment. The procedure was the same as in the preceding experiments, evaluating simulated intestinal absorption with CaCO₂ cells. Dried extract of milk thistle, N-acetyl cysteine and zinc citrate were evaluated. The silicon source was dried extract of bamboo shoots; the Si/Zn content in mg per dose is shown in the table.

**Table 3 Compositions in powder form (mg/dose).**

| | Artichoke, dried extract | Glutathione | Thistle, dried extract. | NAC | Zn⁺⁺ | Si | Silibinin | Cynarin |
|---|---|---|---|---|---|---|---|---|
| S | - | 200 | 200 | - | - | - | 5% | - |
| T | 200 | - | 200 | - | - | 20 | 5% | 40% |
| U | 200 | - | 200 | 200 | | 20 | 5% | 40% |
| V | 200 | 200 | 200 | - | 20 | 20 | 5% | 40% |

The results clearly indicate that the presence of N-acetyl cysteine, silicon and zinc does not interfere with the absorption of cynarin (from dried artichoke extract) or silibinin (from milk thistle extract). They also confirm that the presence of organic silicon promotes intestinal absorption of cynarin.

### Example 5: Orobuccal absorption of multi-ingredient formulations

The procedure was similar to the preceding experiments, evaluating absorption of the active ingredients of artichoke and milk thistle by the oral mucosa, and the possible influence of the other ingredients. The procedure was the same as described in Example 1, using a layer of HOE cells in well systems.

All the compositions comprised 200 mg/dose of dried artichoke extract and 200 mg/dose of dried milk thistle extract. Where present, the source of organic silicon was dried extract of bamboo shoots, and the same compositions without silicon (formulas Or1 and Or2) or comprising inorganic silicon in the form of SiO₂ (formula Or5), were used as reference. L-selenomethionine, equivalent to 60 micrograms of Se per dose, was used as selenium source.

**Table 4 Compositions for orobuccal absorption.**

| | Glutathione | Se | NAC | Zn⁺⁺ | Si | Silibinin | Cynarin |
|---|---|---|---|---|---|---|---|
| Or1 | - | 60 | 200 | - | - | 10% | 10% |
| Or2 | 200 | 60 | - | - | - | 10% | 10% |
| Or3 | - | 60 | 200 | | 20 | 10% | 45% |
| Or4 | 200 | 60 | - | 20 | 20 | 10% | 45% |
| Or5 | 200 | 60 | - | 20 | 20 | 10% | 10% |

A comparison of the results of samples Or3 and Or4 with the same compositions not containing silicon (Or1 and Or2) clearly shows that, as in the case of intestinal absorption, the presence of organic silicon also promotes orobuccal absorption of cynarin, without affecting silibinin absorption. Comparison of samples Or4 and Or5 confirms that silicon must be present in an absorbable form, as also reported in Example 2.

### Example 6: Chewable tablet

| **PHARMACEUTICAL FORM** | **CHEWABLE TABLET 18 mm** | **DOSE** |
|---|---|---|
| **COMPOSITION** | **INGREDIENT** | **mg/tablet** |
| EXCIPIENTS PER TABLET | REDUCED GLUTATHIONE | 250 mg |
| | MILK THISTLE 80% silymarin | 250 mg |
| | VITAMIN E ACETATE | 120 mg |
| | ASCORBIC ACID | 200 mg |
| | VITAMIN D3 100,000 | 20 mg |
| | ZINC citrate 32% | 31.25 mg |
| | SELENOMETHIONINE | 11 mg |
| | ARTICHOKE leaves, dried extract, 2.5% chlorogenic acid | 200 mg |
| | BAMBOO shoots, dried extract, 70% silica | 28.5 mg |
| | | |
| | SACCHARINE | 2.4 mg |
| | SUCRALOSE | 4.8 mg |
| | MANDARIN FLAVOURING IA 009 | 30 mg |
| | ORANGE FLAVOURING IA 230 | 30 mg |
| | SILICON DIOXIDE | 30 mg |
| | FATTY ACID MAGNESIUM SALTS | 20 mg |
| | STABLE FIZZ | 150 mg |
| | XYLITOL (XYLISORB 400) | 400 mg |
| | SORBITOL | 400.55 mg |
| | HYDROXYPROPYL CELLULOSE | 50 mg |
| TOTAL WEIGHT | | 2000 mg |

### Example 7: Chewable tablet

| **PHARMACEUTIC AL FORM** | **CHEWABLE TABLET 18 mm** | **DOSE** |
|---|---|---|
| **COMPOSITION** | **INGREDIENT** | **mg/tablet** |
| EXCIPIENTS PER TABLET | REDUCED GLUTATHIONE | 250 mg |
| | MILK THISTLE 80% silymarin | 250 mg |
| | VITAMIN E ACETATE | 120 mg |
| | ASCORBIC ACID | 200 mg |
| | VITAMIN D3 100,000 | 20 mg |
| | ZINC citrate 32% | 31.25 mg |
| | SELENOMETHIONINE | 11 mg |
| | N-Acetyl cysteine | 200 mg |
| | BAMBOO shoots, dried extract, 70% silica | 28.5 mg |
| | | |
| | SACCHARINE | 2.4 mg |
| | SUCRALOSE | 4.8 mg |
| | MANDARIN FLAVOURING IA 009 | 30 mg |
| | ORANGE FLAVOURING IA 230 | 30 mg |
| | SILICON DIOXIDE | 30 mg |
| | FATTY ACID MAGNESIUM SALTS | 20 mg |
| | STABLE FIZZ | 150 mg |
| | XYLITOL (XYLISORB 400) | 400 mg |
| | SORBITOL | 400.55 mg |
| | HYDROXYPROPYL CELLULOSE | 50 mg |
| TOTAL WEIGHT | | 2000 mg |

### Example 8: Swallowable tablet

| **PHARMACEUTICAL FORM** | **OBLONG SWALLOW ABLE TABLET 21 × 9.9 mm** | **DOSE** |
|---|---|---|
| **COMPOSITION** | **INGREDIENT** | **mg/tablet** |
| | REDUCED GLUTATHIONE | 250.00 mg |
| | MILK THISTLE 80% silymarin | 250.00 mg |
| | VITAMIN E ACETATE | 120.00 mg |
| | ASCORBIC ACID | 200.00 mg |
| | VITAMIN D3 100,000 | 20.00 mg |
| | ZINC citrate 32% | 31.25 mg |
| | SELENOMETHIONINE | 11 mg |
| | ARTICHOKE leaves, dried extract, 2.5% chlorogenic acid | 200.00 mg |
| | BAMBOO shoots, dried extract, 70% silica | 28.50 mg |
| CORE EXCIPIENTS | | |
| | XYLITOL (XYLISORB 400) | 100.00 mg |
| | SORBITOL | 100.00 mg |
| | HYDROXYPROPYL CELLULOSE | 50.00 mg |
| | MICROCRYSTALLINE CELLULOSE | 100.00 mg |
| | SILICON DIOXIDE | 30.00 mg |
| | FATTY ACID MAGNESIUM SALTS | 20.00 mg |
| FILM-COATING EXCIPIENTS | | |
| | OPADRY II WHITE | 30.00 mg |
| | | |
| | | |
| TOTAL WEIGHT | | 1508.75 mg |

### Example 9: Swallowable tablet

| **PHARMACEUTICAL FORM** | **OBLONG SWALLOWABLE TABLET 21 × 9.9 mm** | **DOSE** |
|---|---|---|
| **COMPOSITION** | **INGREDIENT** | **mg/tablet** |
| | REDUCED GLUTATHIONE | 250.00 mg |
| | MILK THISTLE 80% silymarin | 250.00 mg |
| | VITAMIN E ACETATE | 120.00 mg |
| | ASCORBIC ACID | 200.00 mg |
| | VITAMIN D3 100,000 | 20.00 mg |
| | ZINC citrate 32% | 31.25 mg |
| | SELENOMETHIONINE | 11 mg |
| | N-Acetyl cysteine | 200.00 mg |
| | BAMBOO shoots, dried extract, 70% silica | 28.50 mg |
| CORE EXCIPIENTS | | |
| | XYLITOL (XYLISORB 400) | 100.00 mg |
| | SORBITOL | 100.00mg |
| | HYDROXYPROPYL CELLULOSE | 50.00 mg |
| | MICROCRYSTALLINE CELLULOSE | 100.00 mg |
| | SILICON DIOXIDE | 30.00 mg |
| | FATTY ACID MAGNESIUM SALTS | 20.00 mg |
| FILM-COATING EXCIPIENTS | | |
| | OPADRY II WHITE | 30.00 mg |
| | | |
| | | |
| TOTAL WEIGHT | | 1508.75 mg |

### Example 10: Capsule

| **PHARMACEUTICAL FORM** | **CAPSULE TYPE 00** | **DOSE** |
|---|---|---|
| **COMPOSITION** | **INGREDIENT** | **mg/tablet** |
| | REDUCED GLUTATHIONE | 200 mg |
| | MILK THISTLE 80% silymarin | 200 mg |
| | VITAMIN E ACETATE | 120 mg |
| | ASCORBIC ACID | 200 mg |
| | VITAMIN D3 100,000 | 20 mg |
| | ZINC citrate 32% | 31.25 mg |
| | SELENOMETHIONINE | 11 mg |
| | ARTICHOKE leaves, dried extract, 2.5% chlorogenic acid | 100 mg |
| | BAMBOO shoots, dried extract, 70% silica | 28.5 mg |
| CAPSULE TYPE 00 | | |
| | CAPSULE TYPE 00 | 122.4 mg |
| TOTAL WEIGHT | | 1030.15 mg |

### Example 11: capsule

| **PHARMACEUTICAL FORM** | **CAPSULE TYPE 00** | **DOSE** |
|---|---|---|
| **COMPOSITION** | **INGREDIENT** | **mg/tablet** |
| | REDUCED GLUTATHIONE | 200 mg |
| | MILK THISTLE 80% silymarin | 200 mg |
| | VITAMIN E ACETATE | 120 mg |
| | ASCORBIC ACID | 200 mg |
| | VITAMIN D3 100,000 | 20 mg |
| | ZINC citrate 32% | 31.25 mg |
| | SELENOMETHIONINE | 11 mg |
| | N-Acetyl cysteine | 100 mg |
| | BAMBOO shoots, dried extract, 70% silica | 28.5 mg |
| CAPSULE TYPE 00 | | |
| | CAPSULE TYPE 00 | 122.4 mg |
| TOTAL WEIGHT | | 1030.15 mg |

## Claims

1. Dietary or oral pharmaceutical compositions comprising cynarin or an extract containing it, glutathione or a precursor thereof, and a plant extract comprising bioavailable silicon.

2. Compositions according to claim 1 further comprising silybin or an extract containing it, preferably milk thistle extract.

3. Compositions according to claim 1 or 2, wherein the extract comprising cynarin is an artichoke extract, preferably a dried extract of artichoke.

4. Compositions according to one or more of the preceding claims wherein the glutathione takes the form of reduced glutathione, preferably reduced glutathione in crystalline form.

5. Compositions according to one or more of the preceding claims wherein the glutathione precursor is N-acetylcysteine.

6. Compositions according to one or more of the preceding claims wherein the plant extract comprising bioavailable silicon is bamboo shoot extract.

7. Compositions according to one or more of the preceding claims further comprising selenium, preferably L-selenomethionine.

8. Compositions according to one or more of the preceding claims in the form of capsules, tablets, granules, soft gels, solutions, suspensions or syrups.

9. Compositions according to claim 8 in the form of film-coated tablets or orally dissolving granulate.

10. Compositions according to claims 1-9 for use in the treatment of liver disorders.
